# EUROPÄISCHE PATENTANMELDUNG

(11) **EP 1 142 572 A1**
(43) Veröffentlichungstag der Anmeldung: **10.10.2001**
(21) Anmeldenummer: 01107781.5
(22) Anmeldetag: 04.04.2001
(51) Int. Cl.: A61K 31/335, A61P 1/00

(54) **Oxetanon-enthaltende Präparate zur Behandlung von Darmerkrankungen**

(30) Priorität: 04.04.2000 DE 10016667; 04.05.2000 DE 10021618; 19.12.2000 DE 10063413
(71) Anmelder: Helmstädter, Volker, Dr., 69120 Heidelberg (DE)
(72) Erfinder: Helmstädter, Volker, Dr., 69120 Heidelberg (DE)
(74) Vertreter: Boeters, Hans Dietrich, Dr.

(57) **Zusammenfassung**

Die Erfindung betrifft ein pharmazeutisches Präparat oder ein Lebensmittel mit Orlistat oder Lipostatin und dessen Verwendung.

## Beschreibung

In EP 0 129 748 und EP = 189 577 werden (2S, 3S, 5S)-Oxetamone der folgenden Formel beschrieben: 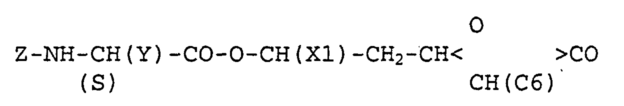 worin bedeuten:
- X1: Undecyl oder (2Z,5Z)-Undecadienyl,
- C6: n-Hexyl,
- Y: Isobutyl und Z Formyl oder
- Y: Carbamoylmethyl und Z Acetyl.

Bei Orlistat bzw. Lipostatin handelt es sich um (2S, 3S, 5S, 7Z, 10Z)-5-[(S)-2-Formamido-4-methyl-valeryloxy]-2-hexyl-3-hydroxy-7,10-hexadecadiensäure-lacton. Die Verwendung von Orlistat-haltigen Präparaten zur Behandlung von Adipositas in Verbindung mit einer leicht kalorienreduzierenden Kost ist bekannt.

Erfindungsgemäß wurde nun festgestellt, daß sich ein Orlistathaltiges Präparat auch zur Therapie chronisch entzündlicher Darmerkrankungen oder zur Therapie von Pouchitis einsetzen läßt. Der erfolgreiche Einsatz für die genannten Indikationen ist um so überraschender, als für die bekannte Verwendung unter anderem folgende Nebenwirkungen behauptet werden: Ölige Flecken am After, Flatulenz mit Stuhlabgang, Stuhlinkontinenz, Abgang öligen Sekrets, vermehrte Stühle, die fettig oder ölig sein können, Bauchschmerzen und Rektumschmerzen.

So betrifft eine Ausführungsform der Erfindung ein pharmazeutisches Präparat zur Therapie chronisch entzündlicher Darmerkrankungen, gekennzeichnet durch einen Gehalt an mindestens einem Oxetamon der vorstehenden Formel neben üblichen Hilfsstoffen und insbesondere mit einem Gehalt an (2S,3S,5S,7Z,10Z)-5-[(S)-2-Formamido-4-methyl-valeryloxy]-2-hexyl-3-hydroxy-7,10-hexadecadiensäure-lacton als Oxetamon.

Das erfindungsgemäße Präparat kann zur Therapie von Morbus Crohn und/oder Colitis ulcerosa vorgesehen sein.

Ferner betrifft die Erfindung ein pharmazeutisches Präparat zur Therapie von Pouchitis bei Patienten mit Colitis ulcerosa nach Colektomie und Anlage eines ileoanalen Pouches, gekennzeichnet durch einen Gehalt an mindestens einem Oxetamon der vorstehenden Formel neben üblichen Hilfsstoffen und insbesondere mit einem Gehalt an (2S,3S,5S,7Z,10Z)-5-[(S)-2-Formamido-4-methyl-valeryloxy]-2-hexyl-3-hydroxy-7,10-hexadecadiensäure-lacton als Oxetamon.

Die erfindungsgemäßen pharmazeutischen Präparate können durch eine orale Applikationsform gekennzeichnet sein.

Ein erfindungsgemäßes Präparat kann dadurch gekennzeichnet sein, daß es als Tablette oder in einer Kapsel vorgesehen ist.

Ferner kann ein erfindungsgemäßes Präparat dadurch gekennzeichnet sein, daß es als Tablette mit magensaftresistentem Überzug vorgesehen ist.

Ferner kann ein erfindungsgemäßes Präparat dadurch gekennzeichnet sein, daß es als Trinklösung vorgesehen ist.

Ferner kann ein erfindungsgemäßes Präparat dadurch gekennzeichnet sein, daß es als Trinklösung vorgesehen werden kann, beispielsweise wenn es als Tablette, Granulat oder Pulver vorliegt.

Ferner kann ein erfindungsgemäßes Präparat dadurch gekennzeichnet sein, daß es als Brausetablette vorliegt.

Eine weitere Ausführungsform der Erfindung betrifft ein Lebensmittel zur Therapie chronisch entzündlicher Darmerkrankungen, gekennzeichnet durch einen Gehalt an mindestens einem Oxetamon der vorstehenden Formel, insbesondere durch einen Gehalt an (2S,3S,5S,7Z,10Z)-5-[(S)-2-Formamido-4-methyl-valeryloxy]-2-hexyl-3-hydroxy-7,10-hexadecadiensäure-lacton.

Eine weitere Ausführungsform der Erfindung betrifft ein Lebensmittel zur Therapie von Pouchitis bei Patienten mit Colitis ulcerosa nach Colektomie und Anlage eines ileoanalen Pouches, gekennzeichnet durch einen Gehalt an mindestens einem Oxetamon der vorstehenden Formel, insbesondere durch einen Gehalt an (2S, 3S, 5S, 7Z, 10Z)-5-[(S)-2-Formamido-4-methylvaleryloxy]-2-hexyl-3-hydroxy-7,10-hexadecadiensäure-lacton.

Eine weitere Ausführungsform der Erfindung betrifft die Verwendung mindestens eines Oxetamons der vorstehenden Formel, insbesondere von (2S, 3S, 5S, 7Z, 10Z)-5-[(S)-2-Formamido-4-methyl-valeryloxy]-2-hexyl-3-hydroxy-7,10-hexadecadiensäurelacton, eines erfindungsgemäßen pharmazeutischen Präparats oder eines erfindungsgemäßen Lebensmittels zur Therapie chronisch entzündlicher Darmerkrankungen und/oder zur Therapie von Pouchitis.

Schließlich betrifft eine Ausführungsform der Erfindung die Verwendung mindestens eines Oxetamons der vorstehenden Formel, insbesondere von (2S,3S,5S,7Z,10Z)-5-[(S)-2-Formamido-4-methyl-valeryloxy]-2-hexyl-3-hydroxy-7,10-hexadecadiensäurelacton zur Herstellung eines erfindungsgemäßen Präparats oder Lebensmittels.

Nachstehend wird die Erfindung durch ein Beispiel näher erläutert.

### Beispiel 1

Es wurden Hartkapseln des Handels verwendet. Gehalt:
Orlistat 120 mg
Mikrokristalline Zellulose
Poly-(O-carboxymethyl)stärke
Natriumsalz
Povidon
Natriumdodecylsulfat
Talkum
Gelatine
Titandioxid

Einem Patienten, der seit 10 Jahren chronisch an Morbus Crohn litt, wurde jeweils eine Kapsel unmittelbar vor dem Frühstück, dem Mittagessen und dem Abendessen täglich 6 Monate lang verabreicht. Danach konnte ein deutlicher Rückgang der chronischen Darmerkrankung bis zur Beschwerdefreiheit diagnostiziert werden.

### Beispiel 2

Es wurden Hartkaspeln des Handels wie in Beispiel 1 verwendet.

Die Behandlung wurde an einem Patienten durchgeführt, de seit über 8 Jahren an einem Morbus Crohn erkrankt und auch im Sinne einer Ileoascendostomie operiert worden war. Zweieinhalb Jahre vor der Behandlung litt der Patient erneut an einem Redidiv des Morbus Crohn im untersten Dünndarm. Dieses Rezidiv hatte bei Beginn der Behandlung bereits zu einer starken Einengung des Darmlumens geführt, so daß zwischenzeitlich auch schon Situationen mit Darmverschluß aufgetreten waren.

Der Patient wurde 6 Monate lang mit dem genannten Präparat bei gleichzeitiger Einnahme von Cortison behandelt. Nachdem die Cortison-Dosierung deutlich reduziert worden war, kam es nicht zu sonst üblichen Entzündungszeichen. Die Behandlung schützte somit vor einem erneuten entzündlichen Schub.

## Patentansprüche

1. Pharmazeutisches Präparat zur Therapie entzündlicher Darmerkrankungen, **gekennzeichnet durch** einen Gehalt an mindestens einem Oxetanon der folgenden Formel: 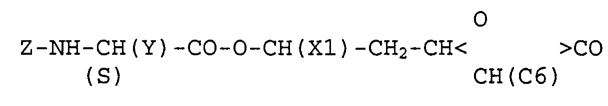 worin bedeuten:
X1 Undecyl oder (2Z,5Z)-Undecadienyl,
C6 n-Hexyl,
Y Isobutyl und Z Formyl oder
Y Carbamoylmethyl und Z Acetyl,
neben üblichen Hilfsstoffen.

2. Pharmazeutisches Präparat zur Therapie chronisch entzündlicher Darmerkrankungen, **gekennzeichnet durch** einen Gehalt an (2S,3S,5S,7Z,10Z)-5-[(S)-2-Formamido-4-methylvaleryloxy]-2-hexyl-3-hydroxy-7,10-hexadecadiensäurelacton neben üblichen Hilfsstoffen.

3. Präparat nach Anspruch 1 oder 2 zur Therapie von Morbus Crohn und/oder Colitis ulcerosa.

4. Pharmazeutisches Präparat zur Therapie von Pouchitis bei Patienten mit Colitis ulcerosa nach Colektomie und Anlage eines ileoanalen Pouches, **gekennzeichnet durch** einen Gehalt an mindestens einem Oxetanon der folgenden Formel: 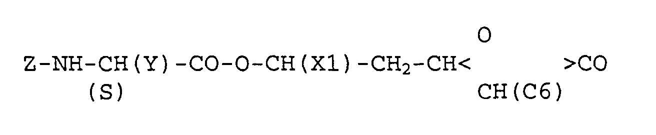 worin bedeuten:
X1 Undecyl oder (2Z,5Z)-Undecadienyl,
C6 n-Hexyl,
Y Isobutyl und Z Formyl oder
Y Carbamoylmethyl und Z Acetyl,
neben üblichen Hilfsstoffen.

5. Pharmazeutisches Präparat zur Therapie von Pouchitis bei Patienten mit Colitis ulcerosa nach Colektomie und Anlage eines ileoanalen Pouches, **gekennzeichnet durch** einen Gehalt an (2S,3S,5S,7Z,10Z)-5-[(S)-2-Formamido-4-methylvaleryl-oxy]-2-hexyl-3-hydroxy-7,10-hexadecadiensäurelacton neben üblichen Hilfsstoffen.

6. Präparat nach einem der vorhergehenden Ansprüche, **gekennzeichnet durch** eine orale Applikationsform.

7. Präparat nach Anspruch 6, **dadurch gekennzeichnet, daß** es als Tablette oder in einer Kapsel vorgesehen ist.

8. Präparat nach Anspruch 6 oder 7, **dadurch gekennzeichnet, daß** es als Retard-Applikationsform vorgesehen ist.

9. Präparat nach Anspruch 8, **dadurch gekennzeichnet, daß** es als Tablette oder Kapsel mit magensaftresistentem Überzug vorgesehen ist.

10. Präparat nach Anspruch 6, **dadurch gekennzeichnet, daß** es als Trinklösung vorgesehen ist.

11. Präparat nach einem der Ansprüche 6 bis 9, **dadurch gekennzeichnet, daß** es als Trinklösung vorgesehen werden kann.

12. Präparat nach Anspruch 11, **dadurch gekennzeichnet, daß** es als Brausetablette vorliegt.

13. Lebensmittel zur Therapie chronisch entzündlicher Darmerkrankungen, **gekennzeichnet durch** einen Gehalt an mindestens einem Oxetanon der folgenden Formel: 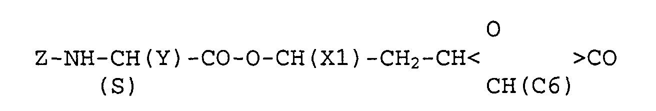 worin bedeuten:
X1 Undecyl oder (2Z,5Z)-Undecadienyl,
C6 n-Hexyl,
Y Isobutyl und Z Formyl oder
Y Carbamoylmethyl und Z Acetyl.

14. Lebensmittel zur Therapie chronisch entzündlicher Darmerkrankungen, **gekennzeichnet durch** einen Gehalt an (2S, 3S, 5S, 7Z, 10Z)-5-[(S)-2-Formamido-4-methylvaleryloxyl]-2-hexyl-3-hydroxy-7,10-hexadecaddien-säurelacton.

15. Lebensmittel zur Therapie von Pouchitis bei Patienten mit Colitis ulcerosa und Colektomie und Anlage eines ileoanalen Pouches, **gekennzeichnet durch** einen Gehalt an mindestens einem Oxetanon der folgenden Formel: 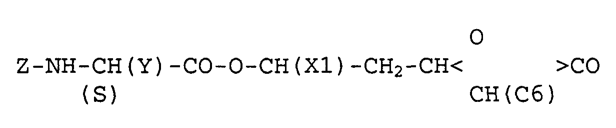 worin bedeuten:
X1 Undecyl oder (2Z,5Z)-Undecadienyl,
C6 n-Hexyl,
Y Isobutyl und Z Formyl oder
Y Carbamoylmethyl und Z Acetyl.

16. Lebensmittel zur Therapie von Pouchitis bei Patienten mit Colitis ulcerosa nach Colektomie und Anlage eines ileoanalen Pouches, **gekennzeichnet durch** einen Gehalt an (2S,3S,5S,7Z,10Z)-5-[(S)-2-Formamido-4-methyl-valeryloxy]-2-hexyl-3-hydroxy-7,10-hexadecadiensäure-lacton.

17. Verwendung mindestens eines Oxetanons der folgenden Formel: 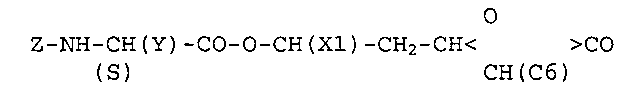 worin bedeuten:
X1 Undecyl oder (2Z,5Z)-Undecadienyl,
C6 n-Hexyl,
Y Isobutyl und Z Formyl oder
Y Carbamoylmethyl und Z Acetyl,
oder eines erfindungsgemäßen pharmazeutischen Präparats oder eines erfindungsgemäßen Lebensmittels gemäß einem der vorhergehenden Ansprüche zur Therapie chronisch entzündlicher Darmerkrankungen und/oder zur Therapie von Pouchitis.

18. Verwendung von (2S,3S,5S,7Z,10Z)-5-[(S)-2-Formamido-4-methyl-valeryloxy]-2-hexyl-3-hydroxy-7,10-hexadecadiensäure-lacton gemäß Anspruch 17.

19. Verwendung mindestens eines Oxetanons der folgenden Formel: 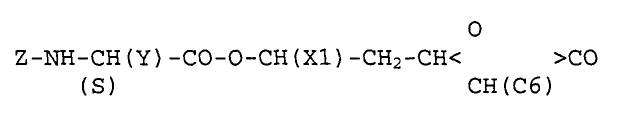 worin bedeuten:
X1 Undecyl oder (2Z,5Z)-Undecadienyl,
C6 n-Hexyl,
Y Isobutyl und Z Formyl oder
Y Carbamoylmethyl und Z Acetyl
zur Herstellung eines Präparats oder Lebensmittels gemäß einem der vorhergehenden Ansprüche.

20. Verwendung von (2S,3S,5S,7Z,10Z)-5-[(S)-2-Formamido-4-methyl-valeryloxy]-2-hexyl-3-hydroxy-7,10-hexadecadiensäure-lacton zur Herstellung eines Präparats oder Lebensmittels gemäß einem der vorhergehenden Ansprüche.
